# EUROPÄISCHE PATENTANMELDUNG

(11) **EP 4 375 360 A1**
(43) Veröffentlichungstag der Anmeldung: **29.05.2024**
(21) Anmeldenummer: 23212033.7
(22) Anmeldetag: 24.11.2023
(51) Int. Cl.: C12M 1/12, B01L 3/00, C12M 1/00, F27B 17/02

(54) **MONTAGEVORRICHTUNG UND MONTAGEVERFAHREN**

(30) Priorität: 24.11.2022 DE 102022131153
(71) Anmelder: TT Innovation AG, 6300 Zug (CH)
(72) Erfinder: Henzmann, Simon Daniel, 4052 Basel (CH); De Maddalena, Tobias, 4303 Kaiseraugst (CH); Huelin, Florian Simon, 5210 Windisch (CH)
(74) Vertreter: Mertzlufft-Paufler, Cornelius

(57) **Zusammenfassung**

Die Erfindung betrifft eine Montagevorrichtung (1), insbesondere zur Montage medizinischer Geräte, die einen mit einem Präparat befüllten Behälter und eine Abgabevorrichtung für das Präparat haben, mit einer Außenbegrenzung (4), durch welche ein Arbeitsraum (6) als ein geschützter Raum (5) definiert ist, und einer Materialdurchführung (7), mit welcher Montagematerial durch eine Öffnung (8) in der Außenbegrenzung (4) transportierbar ist, wobei die Materialdurchführung (7) wenigstens ein Aufnahmeelement (9) aufweist, das zwischen einer Innenposition (10), in welchem ein Aufnahmebereich (12) des wenigstens eine Aufnahmeelements (9) vom Arbeitsraum (6) zugänglich ist, und einer Außenposition (11), in welcher der Aufnahmebereich (12) von außerhalb der Außenbegrenzung (4) zugänglich ist, durch die Öffnung (8) bewegbar ist, wobei das wenigstens eine Aufnahmeelement (9) mit wenigstens einer Verschlusswand (13) gekoppelt ist, welche die Öffnung (8) zumindest in einer Position auf einem Weg (32) des wenigstens einen Aufnahmeelements (9) von der Innenposition (10) zu der Außenposition (11), insbesondere zumindest in der Innenposition (10) und/oder in der Außenposition (11) verschließt, wobei das wenigstens eine Aufnahmeelement (9) eine Lagerposition (14) zwischen der Innenposition (10) und der Außenposition (11) einnehmen kann, in welcher die Öffnung (8) durch die wenigstens eine Verschlusswand (13, 15), vorzugsweise zwei Verschlusswände (13, 15), verschließbar oder verschlossen ist. Die Erfindung betrifft ferner ein Montageverfahren (2)

## Beschreibung

Die Erfindung betrifft eine Montagevorrichtung, insbesondere zur Montage medizinischer Geräte, die einen mit einem Präparat befüllten Behälter und eine Abgabevorrichtung für das Präparat haben, mit einer Außenbegrenzung, durch welche ein Arbeitsraum als ein geschützter Raum definiert ist, und eine Materialdurchführung, mit welcher Montagematerial durch eine Öffnung in der Außenbegrenzung transportierbar ist, wobei die Materialdurchführung wenigstens ein Aufnahmeelement aufweist, dass zwischen einer Innenposition, in welcher ein Aufnahmebereich des wenigstens einen Aufnahmeelements vom Arbeitsraum zugänglich ist, und einer Außenposition, in welcher der Aufnahmebereich von außerhalb der Außenbegrenzung zugänglich ist, durch die Öffnung bewegbar ist.

Derartige Montagevorrichtungen sind insbesondere aus geschützten Räumen der Reinraumtechnik bekannt, welche insbesondere in der pharmazeutischen Industrie im Bereich der aseptischen Herstellung von Arzneimitteln Anwendung finden. Je nach Reinraumklasse kann sich der geschützte Raum beispielsweise in einem RABS (Restricted Access Barrier System), einem Isolator oder einem luft- und mikrobiologisch-kontrolliertem Raum befinden. Ein geschützter Raum kann aber auch ein Arbeitsraum sein, welcher nicht den restriktiven Bedingungen der soeben beschriebenen geschützten Räume unterliegt, sondern welcher aus prozess- und/oder verfahrenstechnischen Gründen durch die Außenbegrenzung abgegrenzt ist von einer weiteren Arbeitsumgebung.

Bei aus der Praxis bekannten Montagevorrichtungen wird das Montagematerial, beispielsweise medizinische Geräte, durch die Öffnung in oder aus dem geschützten Raum transportiert. Meist ist dies ein komplizierter und zeitaufwändiger Prozess, da das Montagematerial durch mehrere, zumeist jeweils manuell zu bedienenden, Verschlusswände bzw. Türen in oder aus dem geschützten Raum bewegt wird. Jeder einzelne Transfer von Montagematerial ist zeitaufwändig, sodass Montageverfahren relativ langsam durchführbar ist.

Die Erfindung betrifft auch ein Montageverfahren, insbesondere zur Montage medizinischer Geräte, die einen mit einem Präparat befüllten Behälter und eine Abgabevorrichtung für das Präparat haben, wobei Montagematerial durch eine Öffnung in einer Außenbegrenzung, die einen Arbeitsraum vorzugsweise als geschützten Raum definiert, gebracht wird, wobei das Montagematerial auf wenigstens einem Aufnahmeelement transportiert wird.

Es ist Aufgabe der Erfindung, Montagevorrichtungen und Montageverfahren zu verbessern. Insbesondere ist es Aufgabe der Erfindung, Montagevorrichtungen so auszugestalten, dass Montageverfahren beschleunigt werden können.

Zur Lösung der genannten Aufgabe sind erfindungsgemäß die Merkmale des Anspruchs 1 vorgesehen. Insbesondere wird somit zur Lösung der genannten Aufgabe bei einer Montagevorrichtung der eingangs beschriebenen Art erfindungsgemäß vorgeschlagen, dass das wenigstens eine Aufnahmeelement mit wenigstens einer Verschlusswand gekoppelt ist, welche die Öffnung zumindest in einer Position auf einem Weg des wenigstens einen Aufnahmeelements von der Innenposition zu der Außenposition, insbesondere zumindest in der Innenposition und/oder in der Außenposition verschließt. Durch die Kopplung der wenigstens einen Verschlusswand an das Aufnahmeelement wird erfindungsgemäß die Öffnung der Materialdurchführung zumindest dann durch die Verschlusswand verschlossen, wenn sich das Aufnahmeelement in der Innen- und oder Außenposition befindet. Somit ist der geschützte Raum zumindest dann von einem Außenbereich durch die Verschlusswand abgegrenzt, wenn das Montagematerial in den Aufnahmebereich eingesetzt oder aus diesen entnommen wird. Vorteilhaft an der erfindungsgemäßen Lösung kann somit sein, dass die Öffnung zum geschützten Raum nur für einen kurzen Zeitraum, insbesondere nur während des Transfers des Montagematerials zwischen Innen- und Außenposition, geöffnet ist. Dies ist insbesondere dann von Vorteil, wenn die Montagevorrichtung sich in einem RABS, einem Isolator und/oder einem luft- und mikrobiologisch-kontrolliertem Raum befindet. Weiterhin kann durch die kurzzeitig geöffnete Öffnung vorteilhaft das Zusammenspiel zwischen automatisierter Montagevorrichtung und dem Eingriff von Menschen oder anderen automatisierten, aber nicht mit der Montagevorrichtung verbundenen Zuführlösungen, verbessert werden, da insbesondere ein Durchgreifen eines Menschen und/oder einer Zufuhrlösung durch die Öffnung verhindert werden kann. Somit werden Sicherheitsrichtlinien verbessert eingehalten. Ferner können Produktionsstopps verhindert werden, welche als Resultat eines Durchgreifens verursacht werden können.

Durch die Kopplung des wenigstens einen Aufnahmeelements mit der wenigstens einen Verschlusswand kann ein Öffnen und/oder Verschließen der Öffnung besonders schnell durchgeführt werden. Somit kann eine Gesamtprozesszeit beschleunigt werden, wodurch eine erfindungsgemäße Montagevorrichtung besonders verbessert ist. Ferner kann durch die Kopplung und dem damit einhergehenden, schnelleren Öffnen und/oder Verschließen der Öffnung ein unerwünschter Übertrag in den geschützten Raum (oder aus dem geschützten Raum hinaus) vermindert oder gar verhindert werden. Beispielsweise kann ein separates Schließen einer Durchreiche nach der Überführung des Aufnahmeelements entfallen. Somit sind Bedienungsfehler vermeidbar.

Die Kopplung des Aufnahmeelements mit der wenigstens einen Verschlusswand kann in vielfältiger Weise, insbesondere wie nachfolgend beschrieben und/oder beansprucht, ausgebildet sein. Insbesondere kann das wenigstens eine Aufnahmeelement direkt mit der Verschlusswand gekoppelt sein, vorzugsweise wie hierin beschrieben. Somit kann das Öffnen und/oder Verschließen besonders schnell durchgeführt werden, sodass eine Gesamtprozesszeit, beispielsweise einer Montage mit anschließender Weiterführung des Montagematerials, beschleunigt sein. Die Kopplung kann auch indirekt sein, sodass vorteilhaft lediglich eine Wirkverbindung zwischen Verschlusswand und Aufnahmeelement besteht. Vorteilhaft an dieser Ausführungsform kann sein, dass eine Konstruktion der Montagevorrichtung vereinfacht und/oder flexibler durchgeführt werden kann.

Ein Vorteil der Kopplung kann auch darin bestehen, dass die wenigstens eine Verschlusswand über ein mit der Kopplung verknüpften Überwachungs- und/oder Kontrollmechanismus nur dann bewegt werden kann, wenn sich das Aufnahmeelement in einer bestimmten Position befindet (oder genau nicht in einer bestimmten Position befindet). So kann beispielsweise ein dem Fachmann an sich bekannter Überwachungs- und/oder Kontrollmechanismus erkennen, dass sich das Aufnahmeelement in der Innenposition befindet, und ferner dafür sorgen, dass die wenigstens eine Verschlusswand (welche nun die Öffnung zu einem Außenbereich hin verschließt, geöffnet werden kann. Somit kann vorteilhaft gewährleistet werden, dass sich die wenigstens eine Verschlusswand (und somit das Aufnahmeelement) nur dann bewegt, wenn dies erwünscht ist.

Die Aufnahmeelemente können dreh- und/oder schwenkbar angeordnet sein, sodass eine Bewegung der Aufnahmeelemente entlang der Materialdurchführung, insbesondere in der Außenposition und/oder Innenposition, flexibel realisiert werden kann. Hierdurch kann die Montagevorrichtung und/oder insbesondere durch die Montagevorrichtung ausführbare Montageverfahren besonders flexibel an die Arbeits- und/oder Arbeitsraumbedingungen angepasst sein.

Die Aufnahmeelemente können herausnehmbar sein. Somit können, beispielsweise in Abhängigkeit der zu montierenden medizinischen Geräte, passgenaue Aufnahmeelemente für das jeweilige medizinische Gerät in die Montagevorrichtung eingesetzt werden. So können beispielsweise Aufnahmeelemente in ihrer Größe an die Größe eines medizinischen Geräts angepasst sein.

Erfindungsgemäß ist ferner vorgesehen, dass das wenigstens eine Aufnahmeelement eine Lagerposition zwischen der Innenposition und der Außenposition einnehmen kann, in welcher die Öffnung durch die wenigstens eine Verschlusswand verschließbar oder verschlossen ist. Somit kann vorteilhaft die Öffnung der Montagevorrichtung auch dann verschlossen werden, wenn das Aufnahmeelement nicht benutzt und/oder wenn das Aufnahmeelement zur Lagerung vom Montagematerial in der Materialdurchführung positioniert wird. Die Lagerposition ermöglich somit vorteilhaft, dass Montagematerial gelagert werden kann, sodass Prozesszeiten verringert werden können. Dies ist ganz besonders von Vorteil, wenn, wie vorgesehen sein kann und hierin beschrieben ist, die Montagevorrichtung mehr als ein Aufnahmeelement hat, insbesondere wenn die Materialdurchführung wenigstens zwei vertikal und/oder horizontal zueinander versetzte Aufnahmeelemente hat, welche unabhängig voneinander bewegbar ausgebildet sind. So kann ein Aufnahmeelement vorteilhaft in der Lagerposition verharren, während das zu diesem Aufnahmeelement versetze Aufnahmeelement in die Innenposition oder Außenposition bewegt werden kann. Die Lagerposition ermöglicht somit, dass insbesondere vertikal oder horizontal von dem Aufnahmeelement, welches sich in Lagerposition befindet, Arbeitsraum geschaffen wird, welcher zu einer Montage genutzt werden kann. Ein derartiger Arbeitsraum kann, wie zuvor bereits eingebracht, ein Raum für die Aufnahme eines zweiten Aufnahmeelements sein, welches sich unabhängig von dem in der Lagerposition befindlichen Aufnahmeelement bewegen kann. Somit ist eine besonders große Flexibilität bei einer Montagevorrichtung erreichbar, was besonders vorteilhaft ist. Somit lässt sich mit zwei, drei oder mehr als drei Aufnahmeelemente, die beispielsweise jeweils wie hierin beschrieben und/oder gleichartig oder identisch zueinander ausgebildet sein können, ein Stapelaufbau realisieren. Beispielsweise können benachbarte Aufnahmeelemente eine Begrenzung nach oben und/oder nach unten für Aufnahmeelemente bilden, insbesondere zur Ausbildung eine Tunnels und/oder zur Aufnahme einer Zwischenposition.

Vorzugsweise kann die Öffnung durch zwei Verschlusswände verschlossen werden, wenn das wenigstens eine Aufnahmeelement eine Lagerposition zwischen der Innenposition und der Außenposition einnimmt. Somit kann ein Verschließen der Materialdurchführung bzw. der Öffnung an zwei Seiten stattfinden, wobei die Lagerposition vorzugsweise zwischen den beiden Verschlusswänden ausgebildet sein kann, beispielsweise in einem wie hierin beschriebenen Schleusenraum. Somit kann Montagematerial besonders vor Einflüssen von außerhalb und innerhalb des geschützten Raums geschützt werden, insbesondere wenn die Materialdurchführung wenigstens zwei vertikal und/oder horizontal zueinander versetzte Aufnahmeelemente hat, welche unabhängig voneinander bewegbar ausgebildet sind.

Bei einer vorteilhaften Ausführungsform kann vorgesehen sein, dass eine erste Verschlusswand der zwei Verschlusswände die Öffnung zumindest in der Außenposition und/oder eine zweite Verschlusswand der zwei Verschlusswände die Öffnung zumindest in der Innenposition verschließt. Von Vorteil bei dieser Ausführungsform kann sein, dass das Verschließen der Öffnung in der Innenposition und der Außenposition jeweils von einer anderen Verschlusswand durchgeführt wird. Somit kann der Verschlussmechanismus der der ersten Verschlusswand von einem Verschlussmechanismus der zweiten Verschlusswand entkoppelt werden. Sodann kann eine Montagevorrichtung besonders flexibel ausgebildet werden.

Alternativ oder zusätzlich kann vorgesehen sein, dass das wenigstens eine Aufnahmeelement zwischen den zwei Verschlusswänden angeordnet ist. Somit kann das wenigstens eine Aufnahmeelement optimal in die Montagevorrichtung eingefasst und durch die zwei Verschlusswände gesichert sein.

Bei einer vorteilhaften Ausführungsform kann vorgesehen sein, dass die wenigstens eine Verschlusswand als eine mitbewegte Verschlusswand an dem wenigstens einen Aufnahmeelement fest angebracht ist. Bei dieser Ausführungsform kann die wenigstens eine Verschlusswand vorteilhaft zusammen mit dem wenigstens einen Aufnahmeelement bewegt werden, so dass vorteilhaft die Öffnung dann verschlossen werden kann, wenn das wenigstens eine Aufnahmeelement die Innenposition, Außenposition und/oder die Lagerposition erreicht. Durch die fest angebrachte Verschlusswand ist ein manuelles Betätigen der Verschlusswand nicht mehr notwendig (und auch nicht mehr möglich), was insbesondere beim pharmazeutischen Industrieumfeld von Vorteil sein kann. Es kann gesagt werden, dass durch das feste Anbringen der wenigstens einen Verschlusswand an dem wenigstens einen Aufnahmeelement ein Öffnen/Verschließen der Öffnung mit einer Bewegung des wenigstens einen Aufnahmeelements entlang der Materialdurchführung synchronisiert werden kann, wodurch eine Gesamtprozesszeit noch weiter verbessert werden kann.

Alternativ oder zusätzlich kann die wenigstens eine Verschlusswand wenigstens an einem Führungselement, insbesondere eines hierin beschriebenen, als mitbewegte Verschlusswand fest angebracht sein. Dies ist insbesondere dann von Vorteil, wenn das wenigstens eine Aufnahmeelement austauschbar angeordnet ist, und die Verschlusswand an der Montagevorrichtung positioniert bleiben soll.

Bei einer vorteilhaften Ausführungsform kann vorgesehen sein, dass die wenigstens eine Verschlusswand die Öffnung, beispielsweise eines Tunnels, in der Außenposition verschließt. Somit kann die Öffnung vorteilhaft verschlossen werden, wenn sich das Aufnahmeelement in der Außenposition befindet. Hierdurch kann verhindert werden, dass durch Manipulationen des Aufnahmeelements und/oder an durch das Aufnahmeelement transportierte oder zu transportierende Montagematerialien Verschmutzungen in die Materialdurchführung und/oder in den geschützten Raum gelangen. Ebenso ist ein manuelles Eingreifen zu einem nicht gewünschten Zeitpunkt verhindert bzw. blockiert.

Vorzugsweise kann vorgesehen sein, dass die wenigstens eine Verschlusswand die Öffnung auch in der Lagerposition verschließt. Somit kann die gleiche Verschlusswand die Öffnung verschließen, wenn sich das Aufnahmeelement in der Außenposition oder in der Lagerposition befindet. Hierdurch können Material und konstruktive Maßnahmen eingespart werden, wodurch eine Montagevorrichtung besonders kundenfreundlich ausgebildet sein kann.

Bei einer vorteilhaften Ausführungsform kann vorgesehen sein, dass die wenigstens eine Verschlusswand als eine stationäre Verschlusswand an einem mit der Außenbegrenzung verbundenen Rahmen beweglich angeordnet ist, wobei die Kopplung mit dem wenigstens einen Aufnahmeelement eine Beweglichkeit der Verschlusswand einschränkt. Beispielsweise kann die stationäre Verschlusswand als Klappe, insbesondere eine wie hierin beschriebene Klappe ausgebildet sein. Dies ist insbesondere von konstruktionellem Vorteil, wenn der Rahmen formschlüssig mit der Außenbegrenzung, beispielsweise als Wandung eines hierin beschrieben RABS, Isolators und/oder eines luft- und mikrobiologisch-kontrolliertem Raums, ausgebildet ist.

Ferner von Vorteil kann die beschränkte Beweglichkeit der Verschlusswand durch die Kopplung der Verschlusswand mit dem Aufnahmeelement für eine Prozesssicherheit sein. Beispielsweise kann sich die Verschlusswand durch den hierin beschriebenen Überwachungs- und/oder Kontrollmechanismus, nur in Abhängigkeit von dem Aufnahmeelement, insbesondere der Position des Aufnahmeelements, bewegen.

Bei einer vorteilhaften Ausführungsform kann vorgesehen sein, dass die wenigstens eine Verschlusswand die Öffnung zumindest in der Innenposition verschließt. Somit kann die Öffnung vorteilhaft verschlossen werden, wenn sich das wenigstens eine Aufnahmeelement in der Innenposition befindet. Hierdurch kann verhindert werden, dass durch Manipulationen des Aufnahmeelements und/oder an durch das Aufnahmeelement transportierte oder zu transportierende Montagematerialien Verschmutzungen in die Materialdurchführung und/oder in Außenbereich gelangen.

Vorzugsweise kann vorgesehen sein, dass die Verschlusswand auch in der Lagerposition die Öffnung verschließt. Somit kann die gleiche Verschlusswand die Öffnung verschließen, wenn sich das wenigstens ein Aufnahmeelement in der Innenposition oder in der Lagerposition befindet. Hierdurch können Material und konstruktive Maßnahmen eingespart werden, wodurch eine Montagevorrichtung besonders kundenfreundlich ausgebildet sein kann.

Bei einer vorteilhaften Ausführungsform kann vorgesehen sein, dass die wenigstens eine Verschlusswand als Klappe ausgebildet ist. Die Klappe kann manuell und/oder automatisch bewegbar sein. Eine als Klappe ausgebildete Verschlusswand ist insbesondere dann von Vorteil, wenn die Klappe als stationäre Verschlusswand ausgebildet ist und/oder wenn die Klappe die Öffnung in der Außenposition verschließt, da somit die Montagevorrichtung konstruktiv und operativ günstig ausgebildet ist.

Bei einer vorteilhaften Ausführungsform kann vorgesehen sein, dass die Verschlusswand eine vorzugsweise mechanische Verriegelung hat, die manuell und/oder automatisiert freigebbar und/oder verschließbar ist. Eine derartige Verriegelung kann eine Prozesssicherheit erhöhen. Durch eine Verriegelung kann beispielsweise ein unerwünschtes Öffnen (oder Verschließen) der Verschlusswand verhindert werden, sodass Arbeiten im geschützten Raum nicht unterbrochen werden. Eine mechanische Verriegelung bietet hierfür eine besonders kosten- und benutzerfreundliche Ausführungsform. Jedoch kann jede andere dem Fachmann bekannte Verrieglung den Kundenwünschen entsprechend zu einer erhöhten Prozesssicherheit beitragen.

Bei einer vorteilhaften Ausführungsform kann vorgesehen sein, dass das wenigstens eine Aufnahmeelement verschieblich angeordnet ist. Somit kann das wenigstens eine Aufnahmeelement vorteilhaft durch die Materialdurchführung hindurch bzw. von der Außenposition über die Lageposition bis hin zur Innenposition verschoben werden (und/oder zurück). Bei dieser bevorzugten Ausführungsform kann das wenigstens eine Aufnahmeelement leicht und mit geringem Zeitaufwand bewegt werden. Insbesondere kann vorteilhaft vorgesehen kann, dass das wenigstens eine Aufnahmeelement durch eine Linearführung verschieblich angeordnet ist.

Von besonders großem Vorteil ist diese Ausführungsform, wenn die wenigstens eine Verschlusswand als eine mitbewegte Verschlusswand an dem Aufnahmeelement fest angebracht ist. Eine derart ausgestaltete Montagevorrichtung ist besonders dazu geeignet, Montageverfahren von medizinischen Geräten, insbesondere wie hierin beschrieben, zu beschleunigen. Separate Schließschritte oder Öffnungsschritte in Bezug auf die Verschlusswand sind somit verzichtbar.

Bei einer vorteilhaften Ausführungsform kann vorgesehen sein, dass das wenigstens eine Aufnahmeelement über Führungselemente zwischen der Innenposition und der Außenposition bewegbar ist. Somit kann vorteilhaft die hierin beschriebene Verschiebung des wenigstens einen Aufnahmeelements über Führungselemente stabil und gesichert durchgeführt werden. Insbesondere vorteilhaft kann sein, wenn die Führungselemente Auszugsschienen bzw. Teleskopschienen sind, wie bevorzugt vorgesehen sein kann, da somit das Öffnen und Verschließen der Öffnung aufgrund reduzierter Reibung verbessert sein kann. Alternativ oder zusätzlich können auch Linearschienen als Führungselemente verwendet werden. Durch die Verwendung von Führungselementen kann insbesondere die hierin beschriebene Linearführung stabil realisiert werden, was eine technische Umsetzung vereinfacht.

Die Führungselemente können so ausgebildet sein, dass sich das wenigstens eine Aufnahmeelement mit oder ohne Auszugsverlust in die Außenposition und/oder Innenposition bewegen lässt. Als Auszugsverlust ist hier beispielsweise der Bereich des wenigstens einen Aufnahmeelements zu verstehen, welcher bei erreichter Außenposition und/oder Innenposition nicht außerhalb der Öffnung ist.

Führungselemente, bei denen das wenigstens eine Aufnahmeelement mit Auszugsverlust in eine Außenposition und/oder Innenposition bewegbar ist, können dann vorteilhaft Verwendung finden, wenn das wenigstens eine Aufnahmeelement besonders groß, insbesondere in Bezug auf die Größe des zu bewegenden Materialguts, ist. Durch den Auszugsverlust kann nämlich vermeidbar sein, dass entlang des Weges unnötig viel Fläche durch das wenigstens eine Aufnahmeelement überstrichen wird.

Führungselemente, bei denen das wenigstens eine Aufnahmeelement ohne Auszugsverlust in eine Außenposition und/oder Innenposition bewegbar ist, können dann vorteilhaft Verwendung finden, wenn die Montagevorrichtung dazu ausgebildet sein soll, wie insbesondere vorteilhaft vorgesehen sein kann, Montagematerial unterschiedlicher Größe bewegen zu können. Durch insbesondere diese Ausführungsform kann eine Montiervorrichtung zur Bearbeitung unterschiedlicher medizinischer Geräte Verwendung finden, was besonders kundenfreundlich ist.

Insbesondere kann vorgesehen sein, dass das wenigstens eine Aufnahmeelement durch die Führungselemente über die oder einer Lagerposition bewegbar ist. Somit kann Montagematerial gesichert und geführt in eine Lagerposition oder aus dieser heraus bewegt werden.

Bei einer vorteilhaften Ausführungsform kann vorgesehen sein, dass das Aufnahmeelement manuell und/oder angetrieben in die Außenposition bewegbar ist. Vorteilhaft bei einem manuell bewegbaren Aufnahmeelement kann sein, dass die Langlebigkeit des Aufnahmeelements erhöht sein kann. Ebenso von Vorteil bei einem manuellen Aufnahmeelement kann sein, dass Wartungen weniger häufig durchgeführt werden müssen. Zudem können durch ein manuell bewegbares Aufnahmeelement Kosten für Antriebe und den damit einhergehenden Kosten für Automatisierungs- und Sicherungsprozesse eingespart werden. Ebenfalls können bei der Verwendung eines manuell bewegbaren Aufnahmeelements Sicherheitsrisiken minimiert werden.

Vorteilhaft bei einem angetriebenen Aufnahmeelement kann sein, dass auf manuelle Arbeit verzichtet werden kann, wodurch eine Bewegung des wenigstens einen Aufnahmeelements in die Außenposition beschleunigt sein kann. Ferner ist diese Ausführungsform vorteilhaft, wenn die Montagevorrichtung mehrere Aufnahmeelemente bewegen soll, insbesondere bei einer gleichzeitigen Bewegung. Ebenso kann diese Ausführungsform von Vorteil sein, wenn mehrerer Aufnahmeelemente vertikal zueinander angeordnet sind, sodass ein Arbeiter Probleme haben kann, hoch angeordnete Aufnahmeelemente in die Außenposition zu bewegen.

Bei einer vorteilhaften Ausführungsform kann vorgesehen sein, dass das Aufnahmeelement mittels einer Antriebseinheit in die Innenposition bewegbar ist. Somit kann ohne manuelles Eingreifen das wenigstens eine Aufnahmeelement in die Innenposition bewegt werden. Dabei kann die Antriebseinheit ein elektrischer Motor, ein Auszugszylinder, beispielsweise ein pneumatischer Zylinder, oder jede andere dem Fachmann bekannte Antriebseinheit sein. Besonders vorteilhaft ist diese Ausführungsform, wenn der geschützte Raum nicht kontaminiert werden soll. Bevorzugt ist diese Ausführungsform, wenn das wenigstens eine Aufnahmeelement durch die hierin beschriebenen Führungselemente bewegbar ist, da somit eine Bewegung des wenigstens einen Aufnahmeelements in die Innenposition besonders akkurat und schnell durchgeführt werden kann, was ein Montageverfahren, insbesondere das hierin beschriebene, verbessert.

Es kann vorgesehen sein, dass das wenigstens eine Aufnahmeelement über die Antriebseinheit mit der wenigstens einen Verschlusswand gekoppelt ist.

Bei einer vorteilhaften Ausführung kann vorgesehen sein, dass ein inneres Führungselement die Bewegung des wenigstens einen Aufnahmeelements in die Lagerposition und/oder Innenposition realisiert und/oder ein äußeres Führungselement die Bewegung des wenigstens einen Aufnahmeelements in die Lagerposition und/oder die Außenposition realisiert. Somit kann vorteilhaft die Bewegung des wenigstens einen Aufnahmeelements in die Innenposition, Lagerposition und in die Außenposition durch unterschiedliche Führungselemente realisiert werden. Dies ist insbesondere dann von Vorteil, wenn die Bewegung des wenigstens einen Aufnahmeelements in die Außenposition manuell durchführbar sein soll und die Bewegung des wenigstens einen Aufnahmeelements in die Innenposition durch eine wie hierin beschriebene Antriebseinheit realisiert werden soll.

Insbesondere kann dabei vorteilhaft vorgesehen sein, dass das äußere Führungselement an dem inneren Führungselement mitbewegbar angeordnet ist. Es kann somit gesagt werden, dass die Bewegungen der Führungselemente gekoppelt werden können, so dass eine gleich geläufige oder gegenläufige Bewegung der Führungselemente vorteilhaft realisierbar ist.

Bei einer vorteilhaften Ausführungsform kann vorgesehen sein, dass das wenigstens eine Aufnahmeelement in seiner Lagerposition in einem abschließbaren oder abgeschlossenen Schleusenraum angeordnet ist. Es kann somit vorgesehen sein, dass die Materialdurchführung als Schleusenraum ausgebildet ist und/oder dass die Materialdurchführung einen Schleusenraum umfasst. Diese Ausführungsform ist insbesondere dann von Vorteil, wenn der geschützte Raum ein RABS, Isolator oder ein luft- und mikrobiologisch-kontrollierter Raum ist. Vorteilhaft an einem Schleusenraum kann sein, dass das in der Lagerposition gehaltene wenigstens eine Aufnahmeelement mitsamt Montagematerial geschützt vor äußeren Einflüssen in dem abgeschlossenen Schleusenraum angeordnet ist. Somit kann das Montagematerial so lange geschützt bzw. gelagert werden, bis eine Weiterverarbeitung, vorzugsweise in dem geschützten Raum, durchgeführt werden soll.

Bei einer erfindungsgemäßen Ausführungsform oder bei einer vorteilhaften Ausführungsform kann vorgesehen sein, dass die Materialdurchführung wenigstens zwei vertikal zueinander versetzte Aufnahmeelemente hat, insbesondere wobei die wenigstens zwei Aufnahmeelemente unabhängig voneinander bewegbar sind. Hierdurch können vorteilhaft mehrere medizinische Geräte und/oder Montagematerialien in einen geschützten Raum (oder daraus hinaus) bewegt werden. Die Bewegung mehrerer Aufnahmeelemente kann versetzt und/oder gleichzeitig geschehen. Es kann ferner vorgesehen sein, dass wenigstens ein Aufnahmeelement in einer Lagerposition verharrt. Ferner kann vorgesehen sein, dass wenigstens ein erstes Aufnahmeelement sich in eine Außenposition bewegt oder darin verharrt und dass wenigstens ein zweites Aufnahmeelement sich in eine Innenposition bewegt oder darin verharrt. Durch die Verwendung wenigstens zwei vorzugweise horizontal und/oder vertikal angeordneter Aufnahmeelemente kann eine Montagevorrichtung besonders variabel und flexibel ausgestalten werden. Insbesondere die Handhabungsvariabilität der Aufnahmeelemente, wie sie zuvor nicht abschließend beschrieben wurde, ist besonders vorteilhaft in der Massenproduktion, da hierbei die Taktung von Montageverfahren, insbesondere wie hierin beschrieben, sehr hoch sein kann

Vorteilhaft bei vertikal angeordneten Aufnahmeelementen kann sein, dass für die Lagerung und/oder Montage von medizinischen Geräten bzw. Montagematerial auf einer vertikalen Achse durchgeführt werden kann. Somit kann der Arbeitsraum effektiv ausgestaltet und genutzt werden. Somit ist eine Montagevorrichtung besonders verbessert.

Bei einer vorteilhaften Ausführungsform kann vorgesehen sein, dass die Materialdurchführung wenigstens zwei vertikal zueinander versetzte Aufnahmeelemente hat. Vorteilhaft insbesondere für Arbeiter bei horizontal angeordneten Aufnahmeelementen kann sein, dass Aufnahmeelemente auf einer einheitlichen Höhe angeordnet sind.

Bei einer bevorzugten Ausführungsform sind wenigstens drei Aufnahmeelemente, besonders bevorzugt wenigstens vier Aufnahmeelemente, horizontal und/oder vertikal angeordnet. Somit kann ein Arbeitsraum besonders effektiv ausgenutzt und gestaltet werden, wobei eine derartige Anordnung ein Montageverfahren vorteilhaft beschleunigen kann, da mehrere Aufnahmeelemente und darin befindliche medizinische Geräte und/oder Montagematerialen nah beieinander positionierbar sind.

Bei einer vorteilhaften Ausführungsform kann vorgesehen sein, dass jedem Aufnahmeelement der wenigstens zwei Aufnahmeelemente eine eigene Öffnung in der Außenbegrenzung zugeordnet ist. Dies ist insbesondere dann von Vorteil, wenn die wenigstens zwei Aufnahmeelemente unabhängig voneinander bewegbar ausgebildet sind, insbesondere wie es hierin beschrieben ist.

Bei einer alternativen oder zusätzlichen Ausführungsform kann vorgesehen sein, dass die Klappe wenigstens zwei Öffnungen abdeckt. Hierdurch kann die Außenbegrenzung zumindest mehrheitlich, besonders bevorzugt ganzheitlich durch die Klappe verschlossen werden. Somit kann eine Außenbegrenzung vorteilhaft durch ein Verschlusselement verschlossen werden, wodurch die Montagevorrichtung günstig ausgebildet sein kann.

Bei einer vorteilhaften Ausführungsform kann vorgesehen sein, dass das wenigstens eine Aufnahmeelement als Schublade ausgebildet ist. Diese Ausführungsform ist besonders vorteilhaft, wenn die Schublade durch Führungselemente, insbesondere die hierin beschriebenen Auszugsschienen und/oder Teleskopschienen, bewegbar ist. Somit kann durch die Bewegung der Schublade weniger Reibung stattfinden, so dass das Aufnahmeelement sehr langlebig ausgebildet sein kann.

Es kann vorgesehen sein, dass die Führungselemente, welche zur Überführung des Aufnahmeelements in die Außenposition fungieren, andersartig ausgebildet sind als die Führungselemente, welche zur Überführung des Aufnahmeelements in die Innenposition fungieren. Somit kann die Ausbildung der Führungselemente kostengünstig und funktionsoptimiert sein. Beispielsweise können kostengünstige Teleskopschienen zur Überführung des Aufnahmeelements in die Außenposition ausgebildet werden und Linearschienen zur Überführung des Aufnahmeelements in die Innenposition. Die Verwendung von Linearschienen kann vorteilhaft insbesondere ein horizontales Spiel des Aufnahmeelements im geschützten Raum minimieren. Somit kann gewährleistet sein, dass sich das Aufnahmeelement bzw. ein bestimmtes Element am und/oder auf dem Aufnahmeelement immer an der gleichen Stelle befindet, wenn das Aufnahmeelement eine Position, beispielsweise die Innenposition, erreicht hat. Hierdurch können Prozessabläufe reproduzierbarer und weniger störanfällig sein.

Vorteilhaft bei einer Schublade kann sein, dass durch die Wände, welche eine Öffnung der Schublade bilden, dass Montagematerial und/oder die medizinischen Geräte sicher halten können. Eine der Wände der Schublade kann eine mitbewegbare, insbesondere wie hierin beschriebene, Verschlusswand bilden, welche den geschützten Raum insbesondere dann nach außen begrenzt, wenn sich die Schublade in der Lagerposition befindet. An diese eine zur Außenbegrenzung ausgebildeten Wand kann auch ein Griffelement befestigt sein, sodass insbesondere bei einer manuellen Bewegung der Schublade dieses Griffelement vorteilhaft gegriffen werden kann. Ein Griffelement kann sich alternativ oder zusätzlich an einer Formateinlage befinden, sodass die Klappe mittels einem Griffelement geöffnet und die Schublade mittels einem zweiten Griffelement an der Formateinlage herausgezogen werden kann.

Alternativ oder zusätzlich kann die Schublade durch Druck auf zur Außenbegrenzung ausgebildeten Wand hin geöffnet werden. Somit kann eine besonders benutzerfreundliche Schublade realisiert werden.

Alternativ oder zusätzlich kann vorgesehen sein, dass ein Boden des wenigstens einen Aufnahmeelements ein herausnehmbares Teil ist. Hierdurch kann der Boden an ein zu transferierendes Montagematerial und/oder medizinisches Gerät angepasst sein. Somit muss nicht ein komplettes Aufnahmeelement aus der Montagevorrichtung herausgenommen werden. Dies ist insbesondere dann von Vorteil, wenn die Montagevorrichtung zur Montage eines andersartig ausgebildeten medizinischen Geräts (im Vergleich zu dem vorherigen medizinischen Gerät) umgestellt werden soll. Diese Ausführungsform kann auch dann vorteilhaft sein, wenn sich ein Aufnahmebereich eines Aufnahmeelements über zwei Etagen der hierin beschriebenen Materialdurchführung erstrecken soll, wobei die Materialdurchführung wenigstens zwei vorzugsweise horizontal und/oder vertikal zueinander versetzte Aufnahmeelemente hat. Somit kann vorteilhaft ein Boden aus einem zweiten Aufnahmeelement entfernt werden, sodass sich ein Aufnahmebereich eines ersten Aufnahmeelements von der ersten Etage in die zweite Etage erstrecken kann.

Diese Umstellung zwischen Produktlinien kann wenigstens ebenso vorteilhaft dadurch realisiert werden, dass das wenigstens eine Aufnahmeelement eine auswechselbare Formateinlage hat, in welche das Montagematerial passgenau einsetzbar ist.

Bei einer vorteilhaften Ausgestaltung kann vorgesehen sein, dass der Weg des wenigstens einen Aufnahmeelements von der Innenposition in die Außenposition durch einen Tunnel führt. Von Vorteil ist dabei, dass zwei voneinander beabstandete Positionen, beispielsweise beide Enden des Tunnels, entlang des Weges definierbar sind, an denen der geschützte Raum verschließbar ist.

Hierbei kann vorgesehen sein, dass eine Länge des Tunnels wenigstens gleich einer Länge des wenigstens einen Aufnahmeelements in Wegrichtung ist. Somit ist im Tunnel eine Lagerposition, insbesondere die bereits erwähnte Lagerposition, definierbar, in welcher das wenigstens eine Aufnahmeelement vollständig einschließbar ist, insbesondere durch zwei Verschlusswände und den Tunnel.

Allgemein kann gesagt werden, dass der Weg beliebig ausgerichtet sein kann, beispielsweise vertikal, horizontal, oder schräg in Bezug auf diese Richtungen. Der Weg kann beispielsweise geradlinig oder einer gebogenen Bahn folgend ausgebildet sein.

Der Weg kann beispielsweise aus mehreren Wegabschnitten, die jeweils geradlinig verlaufen können und/oder zueinander an Übergangspunkten einen Winkel einnehmen und/oder einen Richtungswechsel definieren, zusammengesetzt sein.

Eine horizontale, geradlinige Führung des Weges ist für viele Anwendungsfälle bevorzugt. Sie bietet den Vorteil, dass Bestückung und Entnahme an den Enden des Weges auf gleicher Höhe ablaufen können.

Alternativ oder zusätzlich sind zur Lösung der genannten Aufgabe erfindungsgemäß die Merkmale des nebengeordneten, auf ein Montageverfahren gerichteten Anspruchs vorgesehen. Insbesondere wird somit zur Lösung der genannten Aufgabe bei einem Montageverfahren der eingangs beschriebenen Art erfindungsgemäß vorgeschlagen, dass das wenigstens eine Aufnahmeelement durch die Öffnung verfahren wird, bis die Öffnung verschlossen ist. Somit kann das wenigstens eine Aufnahmeelement in die Innenposition, Außenposition und/oder Lagerposition verfahren werden, bis die Öffnung verschlossen wird. Insbesondere in den vorherig genannten Positionen wird das Montagematerial bearbeitet, in das wenigstens eine oder aus dem wenigstens einen Aufnahmeelement bewegt, oder gelagert, wobei die Öffnung verschlossen ist. Somit kann vorteilhaft bei dem erfindungsgemäßen Montageverfahren ein Luftübertrag zwischen geschütztem Raum und Außenbereich verringert werden. Somit kann ein Kontaminationsübertrag zwischen dem Arbeitsraum und einem Außenraum verringert werden. Dies ist insbesondere dann vorteilhaft, wenn der Arbeitsraum ein geschützter Raum ist, insbesondere ein hierin beschriebener RABS, ein Isolator oder ein luft- und mikrobiologisch-kontrollierter Raum ist. Ferner kann vorteilhaft ein Durchgreifen, beispielsweise durch Entnahmeroboter und/oder Menschen, durch die Öffnung verhindert werden. Hierdurch können Prozessstopps vermieden und Prozessstandards eingehalten werden. Somit kann ein Montageverfahren verbessert werden.

Bei einer vorteilhaften Ausgestaltung des Montageverfahrens kann vorgesehen sein, dass bei dem Montageverfahren eine Montagevorrichtung verwendet wird, wie sie hierin beansprucht ist. Somit kann das Montageverfahren noch weiter verbessert werden, insbesondere durch ein durch die Montagevorrichtung ermöglichten Vorteil.

Die Erfindung wird nun anhand von Ausführungsbeispielen näher beschrieben, ist jedoch nicht auf diese Ausführungsbeispiele beschränkt. Weitere Ausführungsbeispiele ergeben sich durch Kombination der Merkmale einzelner oder mehrerer Ansprüche untereinander und/oder mit einzelnen oder mehreren Merkmalen der Ausführungsbeispiele.

Es zeigen die
- Fig. 1 und 2: ein erfindungsgemäßes Montageverfahren in Seitenansicht (Fig. 1) und Draufsicht (Fig. 2) mit einer erfindungsgemäßen Montagevorrichtung, welche eine mitbewegte Verschlusswand an einem Aufnahmeelement hat,
- Fig. 3 und 4: ein erfindungsgemäßes Montageverfahren in Seitenansicht (Fig. 3) und Draufsicht (Fig. 4) mit einer alternativen erfindungsgemäßen Montagevorrichtung, welche eine mitbewegte Verschlusswand an Führungselementen hat,
- Fig. 5: ein erfindungsgemäßes Montageverfahren in Seitenansicht mit einer alternativen erfindungsgemäßen Montagevorrichtung, welche vertikal zueinander angeordnete Aufnahmeelemente hat, und
- Fig. 6: ein erfindungsgemäßes Montageverfahren in Seitenansicht mit einer alternativen erfindungsgemäßen Montagevorrichtung, wobei das Aufnahmeelement durch ein Führungselement bewegbar ist und ein zweites, vertikal darüber angeordnetes Führungselement mitbewegt wird.

In den gezeigten Figuren sind Montageverfahren 2 zur Montage medizinischer Geräte dargestellt, wobei die medizinischen Geräte einen mit einem Präparat befüllten Behälter und eine Abgabevorrichtung für das Präparat haben. Während der Montageverfahren 2 wird Montagematerial durch eine Öffnung 8 in einer Außenbegrenzung 4, die einen Arbeitsraum 6 als geschützten Raum 5 definiert, gebracht.

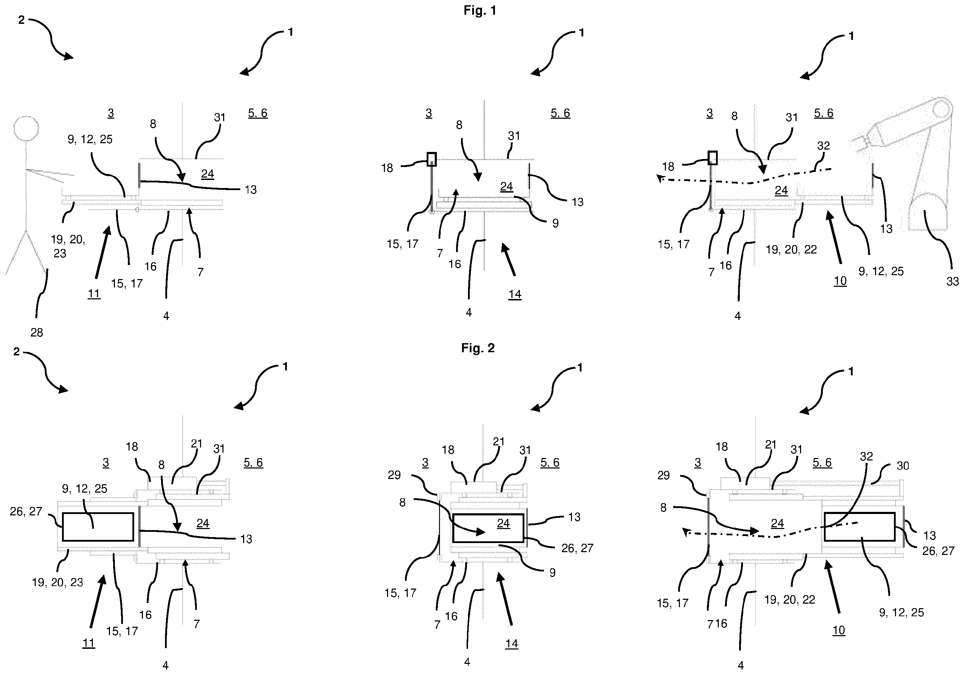

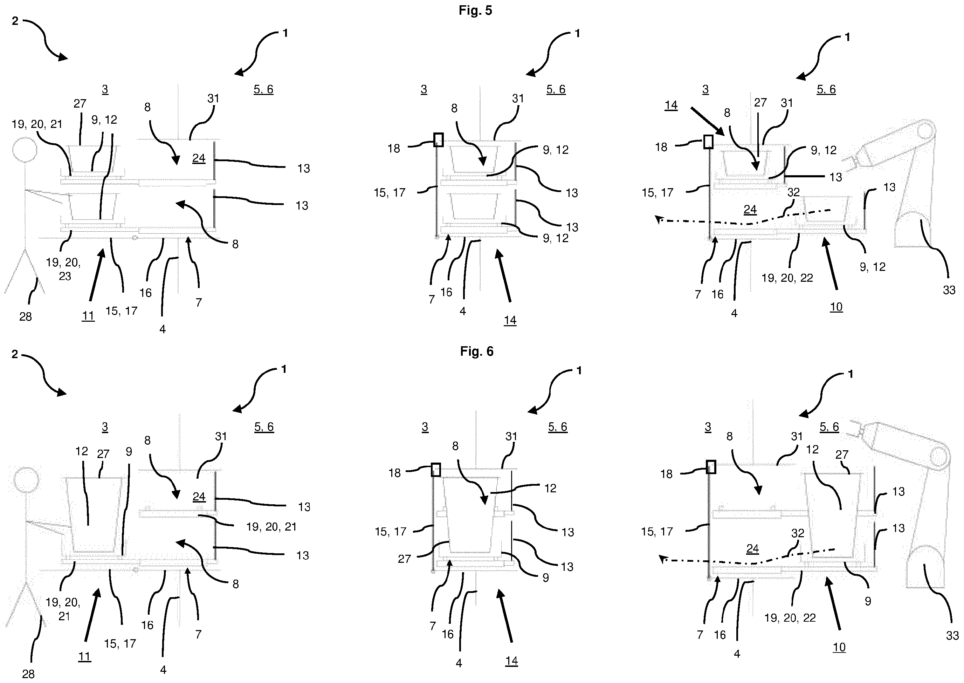

Bei den gezeigten Montageverfahren 2 wird das Montagematerial auf einem Aufnahmeelement 9 (Figuren 1-4, Figur 6) oder auf wenigstens zwei Aufnahmeelementen 9 (Figur 5) transportiert. Die dargestellten Montageverfahren 2 sind dadurch gekennzeichnet, dass das eine oder die wenigstens zwei Aufnahmeelemente 9 durch die Öffnung(en) 8 verfahren werden, bis die Öffnung 8 verschlossen ist.

In den gezeigten Figuren sind drei Positionen gezeigt, in welchen das eine oder die wenigstens zwei Aufnahmeelemente 9 mitsamt Montagematerial (nicht näher gezeigt) verfahren sind. Jeweils auf der linken Seite der einzelnen Figuren befindet sich das eine oder die wenigstens zwei Aufnahmeelemente 9 in der Außenposition 11. Jeweils in den Figuren in der Mitte befinden sich das eine oder die wenigstens zwei Aufnahmeelemente 9 in der Lagerposition 14. Jeweils in den Figuren an der rechten Seite befinden sich das eine oder die wenigstens zwei Aufnahmeelemente 9 in der Innenposition 10, welche sich in dem Arbeitsraum 6, welcher als geschützter Raum 5 ausgebildet ist, befindet. Der geschützte Raum 5,6 der gezeigten Montagevorrichtungen 1 ist als ein luft- und mikrobiologisch-kontrollierter Raum ausgebildet. In weiteren, nicht gezeigten Ausführungsformen können die gezeigten Montageverfahren 2 und/oder Montagevorrichtungen 1 sich in bzw. an einem Isolator oder RABS befinden.

In den Figuren ist zu sehen, dass die Öffnungen 8 der Montagevorrichtungen 1, welche im Nachfolgenden noch näher beschrieben werden, geschlossen sind, wenn die Aufnahmeelemente 9 sich in der Außenposition 11, der Lagerposition 14, oder in der Innenposition 10 befinden.

Das Montageverfahren 2, insbesondere in den gezeigten Ausführungsformen, kann wie folgt ablaufen:
Das Montagematerial und/oder die medizinischen Geräte werden durch einen Arbeiter 28 in einen Aufnahmebereich 12 des Aufnahmeelements 9 der Montagevorrichtungen 1 eingelegt, wenn sich das Aufnahmeelement 9, 12 in der Außenposition 11 befindet (so wie es in den Figuren jeweils in der linken Seite zu sehen ist).

In einem nachfolgenden Schritt wird das Aufnahmeelement 9, 12 mitsamt Montagematerial durch die Öffnung 8 bewegt und in eine Lagerposition 14 überführt (mittlere Darstellungen der Figuren 1 bis 6). Diese Überführung kann manuell durch den Arbeiter 28 durchgeführt werden. In nicht gezeigten Ausführungsformen kann zumindest diese Überführung automatisiert erfolgen, beispielsweise durch eine Antriebseinheit 21. In den gezeigten Ausführungsformen befindet sich die Lagerposition 14 in einem Schleusenraum 24 bzw. einem Tunnel 31, welcher durch eine erste Verschlusswand 13 und einer als Klappe 17 ausgebildeten zweiten Verschlusswand 15 verschlossen ist. Nach erfolgter Überführung des Aufnahmeelements 9, 12 in die Lagerposition 14 wird die Klappe 15, 17 durch eine Verriegelung 18 verschlossen.

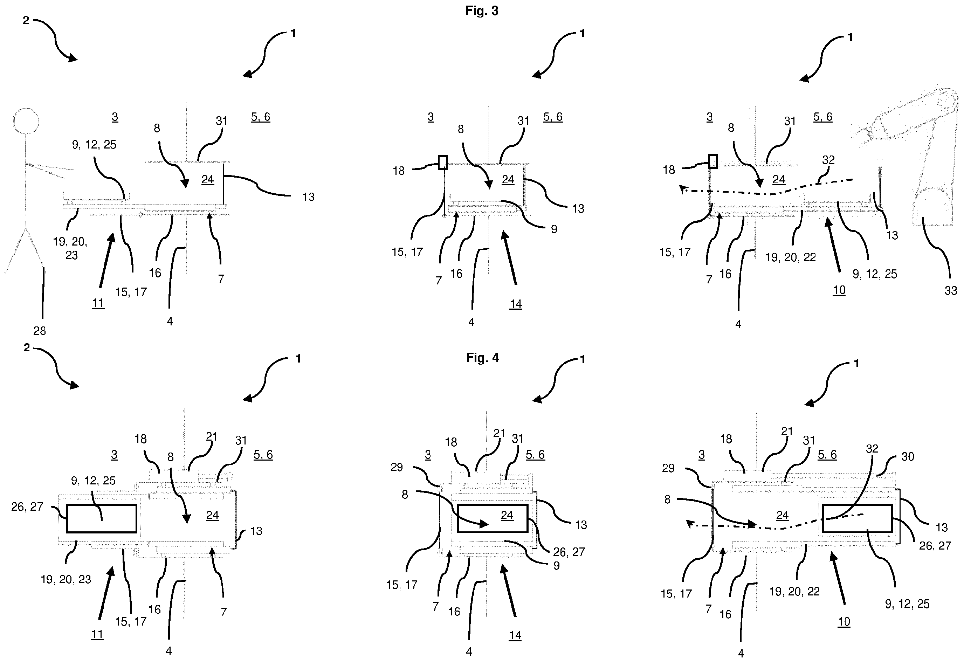

Anschließend wird das Aufnahmeelement 9, 12 mitsamt Montagematerial in den geschützten Raum 5, 6 durch die Materialdurchführung 7 bzw. den Tunnel 31 bis in die Innenposition 10 überführt. Diese Überführung wird durch die Antriebseinheit 21 durchgeführt, welche einen pneumatischen Auszugszylinder 30 umfasst (siehe rechte Abbildungen der Figuren 2 und 4). Dabei kann vorgesehen sein, dass eine Abdeckung des Auszugszylinders 30 bei der Überführung des Aufnahmeelements 9, 12 in den geschützten Raum 5, 6 hinein mit dem Aufnahmeelement 9, 12 gekoppelt ist. Bei der Überführung des Aufnahmeelements 9, 12 in den Außenbereich 3, also durch die Materialdurchführung 7 insbesondere in die Außenposition 11, wird die Kopplung 29 entkoppelt. Von Vorteil bei dieser Ausführungsform ist, dass keine Totzeiten entstehen, sodass eine Gesamtprozesszeit beschleunigt werden kann.

Das Montagematerial und/oder die medizinischen Geräte werden in der Innenposition 10 beispielsweise durch einen Entnahmeroboter 33 entnommen und/oder bearbeitet. Der oder weitere Entnahmeroboter 33 kann/ können alternativ oder zusätzlich als Antriebseinheit 21 fungieren, wodurch ein Überführen des Aufnahmeelements 9 in die Lagerposition 14 und/oder in die Innenposition 14 besonders ressourcensparend ausgeführt werden kann. Durch die Benutzung eines Entnahmeroboters 33 kann auch verhindert werden, dass das Aufnahmeelement 9 bei der Überführung ein zu großes horizontales Spiel erfährt, was Folgeprozesse erschweren kann.

Nach erfolgter Entnahme und/oder Bearbeitung des Montagematerials und/oder der medizinischen Geräte wird das Aufnahmeelement 9, 12 zurück in die Lagerposition 14 und/oder in die Außenposition 11 überführt.

Die gezeigten Montageverfahren 2 sind so realisiert, dass die Klappe 15, 17 nicht geöffnet werden kann, wenn sich das Aufnahmeelement 9 in der Lagerposition 14 und/oder der Innenposition 10 befindet. Somit kann ein Öffnen der Klappe 15, 17 verhindert werden, wenn eine Lagerung und/oder Bearbeitung an dem Aufnahmeelement 9, 12 stattfindet.

Im Folgenden werden die in den Figuren dargestellten Montagevorrichtungen 1 näher beschrieben und verfahrensrelevante Bezüge erläutert.

Die Figuren 1 und 2 zeigen eine erfindungsgemäße Montagevorrichtung 1, welche eine mitbewegte Verschlusswand 13 hat, wobei die mitbewegte Verschlusswand 13 fest an dem Aufnahmeelement 9, 12 befestigt ist.

Das Aufnahmeelement 9, 12 ist als Schublade 25 ausgebildet. Das Aufnahmeelement 9, 12, 25 (ebenso die in den Figuren 3 bis 6 gezeigten Ausführungsformen) hat einen herausnehmbaren Boden 26 und eine auswechselbare Formateinlage 27 (Figuren 2, 4 bis 6), in welche das Montagematerial passgenau einsetzbar ist. Somit können die Montagevorrichtungen 1 bzw. das Montageverfahren 2 an Montagematerial angepasst werden, was besonders nutzerfreundlich ist, da somit verschiedene medizinische Geräte durch eine Vorrichtung montiert werden können.

Alternativ kann vorgesehen sein, dass die Formateinlage 27 einstückig mit dem Aufnahmeelement 9 verbunden ist. Hierdurch kann ein Austausch von Aufnahmeelementen 9, welche insbesondere unterschiedliche Formeinlagen 27 und/oder Böden 26 umfassen, vereinfacht erfolgen.

Die Montagevorrichtung 1 hat eine Materialdurchführung 7, mit welcher das Montagematerial durch die Öffnung 8 in der Außenbegrenzung 4 transportierbar ist, wobei die Materialdurchführung 7 das bereits erwähnte Aufnahmeelement 9, 12, 25 aufweist, dass zwischen der Innenposition 10, der Lagerposition 14 und der Außenposition 11 entlang eines Weges 32, der durch einen Tunnel 31 führt, bewegbar ist. Eine Länge des Tunnels 31, gemessen längs des Weges 32, ist wenigstens gleich einer Länge des Aufnahmeelements 9, gemessen längs des Weges 32, so dass das Aufnahmeelement 9 zumindest in der Lagerposition 14 vollständig von dem Tunnel 31 aufgenommen wird.

Das Aufnahmeelement 9, 12, 25 ist in seinem Aufnahmebereich 12 vom Arbeitsraum 5, 6 her erreichbar, wenn sich das Aufnahmeelement 9, 12, 25 in der Innenposition 10 befindet. Ferner ist das Aufnahmeelement 9, 12, 25 in seinem Aufnahmebereich 12 zugänglich, wenn sich das Aufnahmeelement 9, 12 , 25 in der Außenposition 11 befindet.

Die Außenbegrenzung 4, welche den geschützten Raum 5, 6 definiert, hat eine Öffnung 8, durch welche das Aufnahmeelement 9, 12, 25 bewegbar ist.

Die Montagevorrichtungen 1 dieser (und auch der anderen gezeigten) Ausführungsformen ist dadurch gekennzeichnet, dass das Aufnahmeelement 9, 12, 25 mit der Verschlusswand 13 gekoppelt ist, welche die Öffnung 8 in Außenposition 10 und der Lagerposition 14 verschließt.

In nicht gezeigten Ausführungsformen kann die Verschlusswand 13 die Öffnung 8 alternativ oder zusätzlich dann verschließen, wenn sich das Aufnahmeelement 9, 12 in der Innenposition 10 befindet.

Wenn sich das Aufnahmeelement 9, 12, 25 in der Innenposition 10 befindet, wird die Öffnung 8 durch die Klappe 15, 17 verschlossen. Die bewegliche Klappe 15, 17 ist stationär mit einem Rahmen 16 verbunden, welcher an der Außenbegrenzung 4 angeordnet ist. Die Klappe 15, 17 ist durch die bereits beschrieben Kopplung 29 mit dem Aufnahmeelement 9, 12, 25 verbunden, welche eine Beweglichkeit der Verschlusswand 15 so einschränkt, dass die Klappe 15, 17 nicht geöffnet werden kann, wenn sich das Aufnahmeelement 9, 12, 25 in der Lagerposition 14 und/oder in der Innenposition 10 befindet. Die somit verriegelte Klappe 15, 17 kann durch die zwischen Lagerposition 14 und Außenposition 15 angeordnete Verriegelung 18 manuell und/oder automatisiert freigegeben (und verschlossen) werden (siehe Figuren 2 und 4).

Das Aufnahmeelement 9, 12, 25 ist über Führungselemente 19, welche als Auszugsschienen/ Teleskopschienen 20 ausgebildet sind, zwischen der Innenposition 10 und der Außenposition 11, über die Lagerposition 14 hin, bewegbar. Dabei ist die Bewegung des Aufnahmeelements 9, 12, 25 in den geschützten Raum 5, 6 hinein angetrieben durch die Antriebseinheit 21, 30. Die Bewegung des Aufnahmeelements 9, 12, 25 in Richtung Außenbereich 3 ist manuell und wird durch den Arbeiter 28 bewerkstelligt.

In einer nicht gezeigten Ausführungsform kann vorgesehen sein, dass die Bewegung des Aufnahmeelements 9, 12 in den Außenbereich 3 hin angetrieben ist, und die Bewegung in den geschützten Raum 5, 6 manuell erfolgt.

In weiteren nicht gezeigten Ausführungsformen können alle Bewegungen des Aufnahmeelements 9, 12 manuell oder angetrieben sein.

In der gezeigten Ausführungsform (Fig. 1 und 2) realisiert ein inneres Führungselement 22 die Bewegung in die Innenposition 10 und ein äußeres Führungselement 23 die Bewegung in die Außenposition 11.

Die Ausführungsformen der Figuren 3 bis 6 zeigen alternative Montagevorrichtungen 1. Funktionell und/oder konstruktiv zu dem zuvor beschriebenen Ausführungsbeispiel gleichartige oder identische Bauteile und Funktionseinheiten sind mit denselben Bezugszeichen bezeichnet und nicht noch einmal gesondert beschrieben. Die Ausführungen zu den Figuren 1 bis 2 gelten daher zu den Figuren 3 bis 6 entsprechend.

Das Ausführungsbeispiel nach den Figuren 3 und 4 unterscheidet sich von dem Ausführungsbeispiel der Figuren 1, 2 zumindest dadurch, dass die Verschlusswand 13 verschiebbar an den Führungselementen 19, 20, 22 angeordnet ist. Dies ist besonders vorteilhaft, da das Aufnahmeelement 9, 12, 25 austauschbar angeordnet ist und somit die Verschlusswand 13 an der Montagevorrichtung 1 unabhängig von dem Aufnahmeelement 9 befestigt oder befestigbar ist.

Das Ausführungsbeispiel nach Figur 5 unterscheidet sich von dem Ausführungsbeispielen der Figuren 1 bis 4 zumindest dadurch, dass die Materialdurchführung 7 zwei vertikal zueinander versetzte Aufnahmeelemente 9, 12 hat, wobei die zwei Aufnahmeelemente 9, 12 unabhängig voneinander bewegbar sind (Figur 6, rechte Seite). Jedes der beiden Aufnahmeelemente 9, 12 hat eine eigene Öffnung 8 in der Außenbegrenzung 4 zugeordnet. Ferner deckt die Klappe 15, 17 die zwei Öffnungen 8 ab, sodass kostengünstig nur eine Verschlusswand 15, 17 benötigt wird.

In nicht gezeigten Ausführungsformen hat die Materialdurchführung 7 mehr als zwei zueinander versetzte Aufnahmeelemente 9, 12, welche je nach Ausführungsform horizontal und/oder vertikal zueinander angeordnet sind. Jedes dieser Aufnahmeelemente 9, 12 ist eine eigene Öffnung 8 in der Außenbegrenzung 4 zugeordnet.

Das Ausführungsbeispiel nach Figur 6 unterscheidet sich von den Ausführungsbeispielen der Figuren 1 bis 5 zumindest dadurch, dass das Aufnahmeelement 9, 12 durch zwei Öffnungen 8, welche vertikal angeordnet sind, durch die Materialdurchführung 7 überführt wird bzw. überführbar ist. Somit können Aufnahmeelemente 9 mit besonders großen Aufnahmebereichen 12 bei Montageverfahren 2 verwendet werden, um besonders große medizinische Geräte zu montieren.

Die Erfindung betrifft eine Montagevorrichtung 1, insbesondere zur Montage medizinischer Geräte, die einen mit einem Präparat befüllten Behälter und eine Abgabevorrichtung für das Präparat haben, mit einer Außenbegrenzung 4, durch welche ein Arbeitsraum 6 als ein geschützter Raum 5 definiert ist, und einer Materialdurchführung 7, mit welcher Montagematerial durch eine Öffnung 8 in der Außenbegrenzung 4 transportierbar ist, wobei die Materialdurchführung 7 wenigstens ein Aufnahmeelement 9 aufweist, das zwischen einer Innenposition 10, in welchem ein Aufnahmebereich 12 des wenigstens einen Aufnahmeelements 9 vom Arbeitsraum 6 zugänglich ist, und einer Außenposition 11, in welcher der Aufnahmebereich 12 von außerhalb der Außenbegrenzung 4 zugänglich ist, durch die Öffnung 8 bewegbar ist, wobei das wenigstens eine Aufnahmeelement 9 mit wenigstens einer Verschlusswand 13 gekoppelt ist, welche die Öffnung 8 zumindest in einer Position auf einem Weg 32 des wenigstens einen Aufnahmeelements 9 von der Innenposition 10 zu der Außenposition 11, insbesondere zumindest in der Innenposition 10 und/oder in der Außenposition 11 verschließt, wobei das wenigstens eine Aufnahmeelement 9 eine Lagerposition 14 zwischen der Innenposition 10 und der Außenposition 11 einnehmen kann, in welcher die Öffnung 8 durch die wenigstens eine Verschlusswand 13, 15, vorzugsweise zwei Verschlusswände 13, 15, verschließbar oder verschlossen ist. Die Erfindung betrifft ferner ein Montageverfahren 2.

### Bezugszeichenliste

- 1: Montagevorrichtung
- 2: Montageverfahren
- 3: Außenbereich
- 4: Außenbegrenzung
- 5: geschützter Raum
- 6: Arbeitsraum
- 7: Materialdurchführung
- 8: Öffnung
- 9: Aufnahmeelement
- 10: Innenposition
- 11: Außenposition
- 12: Aufnahmebereich
- 13: erste Verschlusswand
- 14: Lagerposition
- 15: zweite Verschlusswand
- 16: Rahmen
- 17: Klappe
- 18: Verriegelung
- 19: Führungselemente
- 20: Auszugsschienen / Teleskopschienen
- 21: Antriebseinheit
- 22: inneres Führungselement
- 23: äußeres Führungselement
- 24: Schleusenraum
- 25: Schublade
- 26: Boden
- 27: Formateinlage
- 28: Arbeiter
- 29: Kopplung
- 30: Auszugszylinder
- 31: Tunnel
- 32: Weg
- 33: Entnahmeroboter

## Patentansprüche

1. Montagevorrichtung (1), insbesondere zur Montage medizinischer Geräte, die einen mit einem Präparat befüllten Behälter und eine Abgabevorrichtung für das Präparat haben, mit einer Außenbegrenzung (4), durch welche ein Arbeitsraum (6) als ein geschützter Raum (5) definiert ist, und einer Materialdurchführung (7), mit welcher Montagematerial durch eine Öffnung (8) in der Außenbegrenzung (4) transportierbar ist, wobei die Materialdurchführung (7) wenigstens ein Aufnahmeelement (9) aufweist, das zwischen einer Innenposition (10), in welchem ein Aufnahmebereich (12) des wenigstens einen Aufnahmeelements (9) vom Arbeitsraum (6) zugänglich ist, und einer Außenposition (11), in welcher der Aufnahmebereich (12) von außerhalb der Außenbegrenzung (4) zugänglich ist, durch die Öffnung (8) bewegbar ist dass das wenigstens eine Aufnahmeelement (9) mit wenigstens einer Verschlusswand (13, 15) gekoppelt ist, welche die Öffnung (8) zumindest in einer Position auf einem Weg (32) des wenigstens einen Aufnahmeelements (9) von der Innenposition (10) zu der Außenposition (11), insbesondere zumindest in der Innenposition (10) und/oder in der Außenposition (11), verschließt, **dadurch gekennzeichnet, dass** das wenigstens eine Aufnahmeelement (9) eine Lagerposition (14) zwischen der Innenposition (10) und der Außenposition (11) einnehmen kann, in welcher die Öffnung (8) durch die wenigstens eine Verschlusswand (13, 15), vorzugsweise zwei Verschlusswände (13, 15), verschließbar oder verschlossen ist.

2. Montagevorrichtung (1) nach Anspruch 1, **dadurch gekennzeichnet, dass** das wenigstens eine Aufnahmeelement (9) zwischen den zwei Verschlusswänden (13, 15) angeordnet ist und/oder eine erste Verschlusswand (13) der zwei Verschlusswände (13, 15) die Öffnung (8) zumindest in der Außenposition (11) und eine zweite Verschlusswand (15) der zwei Verschlusswände (13, 15) die Öffnung (8) zumindest in der Innenposition (10) verschließt.

3. Montagevorrichtung (1) nach einem der vorangehenden Ansprüche, **dadurch gekennzeichnet, dass** die wenigstens eine Verschlusswand (13) als eine mitbewegte Verschlusswand (13) an dem wenigstens einen Aufnahmeelement (9) fest angebracht ist und/oder dass die mitbewegte Verschlusswand (13) an wenigstens einem Führungselement (19) fest angebracht ist.

4. Montagevorrichtung (1) nach einem der vorangehenden Ansprüche, **dadurch gekennzeichnet, dass** die wenigstens eine Verschlusswand (13, 15) die Öffnung (8) in der Außenposition (11) verschließt, vorzugsweise auch in der Lagerposition (14), und/oder Montagevorrichtung (1) nach einem der vorangehenden Ansprüche, **dadurch gekennzeichnet, dass** die wenigstens eine Verschlusswand (13, 15) die Öffnung (8) zumindest in der Innenposition (10) verschließt, vorzugsweise auch in der Lagerposition (14).

5. Montagevorrichtung (1) nach einem der vorangehenden Ansprüche, **dadurch gekennzeichnet, dass** die wenigstens eine Verschlusswand (13, 15) als eine stationäre Verschlusswand (13, 15) an einem mit der Außenbegrenzung (4) verbundenen Rahmen (16) beweglich angeordnet ist, wobei die Kopplung (29) mit dem wenigstens einen Aufnahmeelement (9) eine Beweglichkeit der Verschlusswand (13, 15) einschränkt.

6. Montagevorrichtung (1) nach einem der vorangehenden Ansprüche, **dadurch gekennzeichnet, dass** die wenigstens eine Verschlusswand (13, 15) als Klappe (17) ausgebildet ist, und/oder dass die wenigstens eine Verschlusswand (13, 15), insbesondere zwischen der Lagerposition (14) und der Außenposition (11), eine vorzugsweise mechanische Verriegelung (18) hat, die manuell und/oder automatisiert freigebbar und / oder verschließbar ist

7. Montagevorrichtung (1) nach einem der vorangehenden Ansprüche, **dadurch gekennzeichnet, dass** das wenigstens eine Aufnahmeelement (9) verschieblich, vorzugsweise durch eine Linearführung, angeordnet ist, und/oder dass das wenigstens eine Aufnahmeelement (9) über Führungselemente (19), vorzugsweise Auszugsschienen / Teleskopschienen (20) zwischen der Innenposition (10) und der Außenposition (11), insbesondere über die oder eine Lagerposition (14), bewegbar ist.

8. Montagevorrichtung (1) nach einem der vorangehenden Ansprüche, **dadurch gekennzeichnet, dass** das wenigstens eine Aufnahmeelement (9) manuell und / oder angetrieben in die Außenposition (11) bewegbar ist, und/oder dass das wenigstens eine Aufnahmeelement (9) mittels einer Antriebseinheit (21) in die Innenposition (10) bewegbar ist.

9. Montagevorrichtung (1) nach einem der vorangehenden Ansprüche, **dadurch gekennzeichnet, dass** ein inneres Führungselement (22) die Bewegung des wenigstens einen Aufnahmeelements (9) in die Lagerposition (14) und/oder die Innenposition (10) realisiert und/oder ein äußeres Führungselement (23) die Bewegung des wenigstens einen Aufnahmeelements (9) Lagerposition (14) und/oder in die Außenposition (11) realisiert, insbesondere wobei das äußere Führungselement (23) an dem inneren Führungselement (22) mitbewegbar angeordnet ist.

10. Montagevorrichtung (1) nach einem der vorangehenden Ansprüche, **dadurch gekennzeichnet, dass** das wenigstens eine Aufnahmeelement (9) in seiner Lagerposition (14) in einem abschließbaren oder abgeschlossenen Schleusenraum (24) angeordnet ist.

11. Montagevorrichtung nach dem Oberbegriff von Anspruch 1 oder nach einem der vorangehenden Ansprüche, **dadurch gekennzeichnet, dass** die Materialdurchführung (1) wenigstens zwei, vorzugsweise drei oder mehr als drei, vertikal zueinander versetzte Aufnahmeelemente (9) hat, insbesondere wobei die wenigstens zwei Aufnahmeelemente (9) unabhängig voneinander bewegbar sind.

12. Montagevorrichtung (1) nach einem der vorangehenden Ansprüche, **dadurch gekennzeichnet, dass** die Materialdurchführung (7) wenigstens zweihorizontal zueinander versetzte Aufnahmeelemente (9) hat, insbesondere wobei die wenigstens zwei Aufnahmeelemente (9) unabhängig voneinander bewegbar sind.

13. Montagevorrichtung (1) nach einem der vorangehenden Ansprüche, **dadurch gekennzeichnet, dass** jedem Aufnahmeelement (9) der wenigstens zwei Aufnahmeelemente (9) eine eigene Öffnung (8) in der Außenbegrenzung (4) zugeordnet ist, und/oder dass die Klappe (17) wenigstens zwei Öffnungen (8) abdeckt.

14. Montagevorrichtung (1) nach dem vorangehenden Anspruch,
**dadurch gekennzeichnet, dass** wenigstens eine Aufnahmeelement (9) als Schublade (25) ausgebildet ist und/oder dass ein Boden (26) des wenigstens einen Aufnahmeelements (9) ein herausnehmbares Teil ist, und/oder dass das wenigstens eine Aufnahmeelement (9) eine vorzugsweise auswechselbare Formateinlage (27) hat, in welche das Montagematerial passgenau einsetzbar ist.

15. Montagevorrichtung (1) nach einem der vorangehenden Ansprüche, **dadurch gekennzeichnet, dass** der Weg (32) des wenigstens einen Aufnahmeelements (9) von der Innenposition (10) in die Außenposition (11) durch einen Tunnel (31) führt, insbesondere wobei eine Länge des Tunnels (31) wenigstens gleich einer Länge des wenigstens einen Aufnahmeelements (9) in Wegrichtung ist.

16. Montageverfahren (2), insbesondere zur Montage medizinischer Geräte, insbesondere unter Verwendung einer Montagevorrichtung (1) nach den Ansprüchen 1 bis 15, die einen mit einem Präparat befüllten Behälter und eine Abgabevorrichtung für das Präparat haben, wobei Montagematerial durch eine Öffnung (8) in einer Außenbegrenzung (4), die einen Arbeitsraum (6) vorzugsweise als geschützten Raum (5) definiert, gebracht wird, wobei das Montagematerial auf wenigstens einem Aufnahmeelement (9) transportiert wird, **dadurch gekennzeichnet, dass** das wenigstens eine Aufnahmeelement (9) durch die Öffnung (8) verfahren wird, bis die Öffnung (8) verschlossen ist.
